# EUROPEAN PATENT SPECIFICATION

(11) **EP 1 827 340 B1**
(45) Date of publication and mention of the grant of the patent: **31.10.2012**
(21) Application number: 04809123.5
(22) Date of filing: 21.12.2004
(51) Int. Cl.: A61F 13/00, A61F 5/48

(54) **ABSORBENT PAD**
SAUGFÄHIGES POLSTER
MATELAS ABSORBANT

(43) Date of publication of application: 05.09.2007
(73) Proprietor: SCA Hygiene Products AB, 405 03 Göteborg (SE)
(72) Inventor: YAZARLO, Shadi, S-431 36 Mölndal (SE); HERMANSSON, Sofia, S-426 71 Västra Frölunda (SE)
(74) Representative: Börlin, Maria
(86) International application number: PCT/SE2004/001950
(87) International publication number: WO 2006/068544

(56) References cited:
- EP-A2- 0 815 820
- WO-A1-01/03842
- US-A1- 2003 082 966
- US-A1- 2003 121 101

## Description

### TECHNICAL FIELD

The present invention relates to a pad of the disposable type for the protection of objects that require to be protected, for example seat cushions, bedclothes or changing tables. The pad comprises a liquid-receiving covering layer intended to face away from the object that requires to be protected and an essentially liquid-tight backing layer intended to face towards the object that requires to be protected when it is being used. The pad also comprises means for closing the pad after use.

### BACKGROUND

In hospitals and other care institutions, and in the area of care in the home, different kinds of pad of the disposable type are used, such as chair or bed protectors, to prevent urine and/or feces from penetrating into and contaminating bedclothes and mattresses in beds and seat cushions on chairs US 2003/0082966 A1 discloses such a pad. It is not uncommon, when changing infants' diapers, for pads to be used, for example in order to protect the changing table on which the diaper change is performed. When a pad of this kind has become contaminated with urine and/or feces, the normal procedure is for the pad to be folded together or rolled up with the liquid-tight backing layer facing outwards, as a consequence of which the contents of the protector are well-shielded from the surroundings. Such handling of the pad facilitates its subsequent handling and reduces problems of unpleasant odors when disposal of the pad is necessary.

The situation also arises in which a used diaper is placed on the pad, after which the pad is folded around the diaper so that a hygienic package is formed that is suitable for disposal.

One type of pad intended for changing tables is described in document JP 2002248126 A. The pad includes an adhesive surface arranged on the backing layer adjacent to one of the edges of the protector, in conjunction with which the adhesive surface is intended to be used for closing the pad after it has been used and folded together. The document also stipulates that it is appropriate for the used diaper to be placed inside the protector before it is folded up and disposed of.
One disadvantage of providing the protector with an adhesive surface directly on the backing layer is that the adhesive surface faces outwards away from the folded protector when the latter is folded/rolled up with the backing later facing outwards, which is the best and most commonly encountered method of folding. The part of the pad that contains the adhesive surface must accordingly be folded one more time in order to cause the adhesive surface to face towards the backing layer of the pad so that closure of the pad can take place.
The joint between the backing layer and the adhesive surface will also be loaded in an unfavorable fashion, because of the above-mentioned folding, and will be subjected to a splitting force which reduces the strength of the joint significantly compared with the situation in which the joint is instead subjected to a shearing force.
A further disadvantage of an adhesive surface arranged in accordance with that described in JP 2002248126 A is that the adhesive surface must be covered with some form of protective layer before the adhesive surface is used, as a consequence of which the protective layer must be removed before use. The separate protective layer thus requires to be handled in conjunction with disposal of the pad, which can present certain problems.
Other documents describe different types of pad comprising means for securing the pad to a bed, for example, when it is being used. Document US 4,097,943, for example, describes a bed pad comprising adhesive strings along the edges for the purpose of securing it to the bedclothes.
In principle the same problem is encountered with this type of protector, as with the protector described in document JP 2002248126 A.

Described in document EP 0,218,568 are pads provided with special surfaces comprising high-friction materials, in which the intention of the high-friction surfaces is to cause the pad to lie still in the bed even if the patient lying in the bed moves. The function of protectors of this kind essentially does not take account of the closure of the protector in a folded configuration that is suitable for disposal after use.

The need exists, therefore, for a pad that is capable of being folded and closed in a simple and hygienic fashion after it has been folded together or rolled up in conjunction with the intended disposal of the pad.

### DESCRIPTION OF THE INVENTION

A pad of the kind referred to by way of introduction has been made available with the present invention, however, which essentially overcomes the problems that were associated with earlier previously disclosed articles.

A pad in accordance with the invention is characterized primarily in that the means for closing the pad after use comprises at least one securing flap comprising a first end area and a second end area. The first end area is permanently attached to the backing layer or to the liquid-receiving covering layer, and the second end area of the securing flap comprises a securing device intended to be secured to the pad after use.
A protective layer is arranged between the securing device and the backing layer or the liquid-receiving covering layer. The protective layer is treated with a release agent on the side which faces towards the securing device, and in conjunction with which the opposite side of the protective layer is permanently attached to the backing layer or to the liquid-receiving covering layer.
By providing the pad with a securing flap, where one end is permanently attached to the pad and the other end is free and comprises a securing device, the securing flap constitutes an effective closing device for the pad after it has been used and may possibly contain urine.

In accordance with one embodiment, the pad comprises an absorbent layer arranged between the liquid-receiving covering layer and the backing layer. By providing the pad with a separate, absorbent layer that is separated from the liquid-receiving layer, quite large quantities of matter excreted from the body can be absorbed without the pad having to be unreasonably costly to manufacture.

In accordance with one embodiment of the pad, the securing device is arranged on the side of the securing flap that faces towards the pad. The securing flap can be closed, by positioning the securing device against the pad, without the securing flap requiring to be rotated about its longitudinal direction.

In accordance with one embodiment, the securing device comprises an adhesive surface. Since by far the majority of pads exhibit a backing layer made of plastic film, to which adhesive can be attached in an excellent fashion, a securing device comprising adhesive offers both an inexpensive and an effectively functioning solution.

In accordance with one embodiment, the means for closing the pad is arranged on the backing layer of the pad. The application of the means for closing arranged on the backing layer of the pad means that the means does not interfere with the use of the pad, for example as a bed protector or a changing table protector. The application further means that it is particularly easy to use the means after the pad has been folded together/rolled up with the essentially liquid-tight layer facing outwards, because the means automatically ends up on the outside in conjunction with such folding together/rolling up.

In accordance with one embodiment, the securing flap consists of a paper strip, in conjunction with which the paper strip includes a folded area so that the securing flap can be extended by stretching out the folded area. Paper is an inexpensive and effectively functioning material for a securing flap that is suitable as a closing device for an pad.

In accordance with another embodiment of the invention, the securing flap comprises an elastic area, in conjunction with which the securing flap can be extended by stretching out the elastic area.

In accordance with one embodiment, the second end area of the securing flap is arranged between the first end area of the securing flap and the edge of the pad when the securing flap is in its first folded or unstretched state. When the fold is unfolded or when the elastic area is stretched out, the second end area of the securing flap reaches beyond the edge of the pad. This embodiment permits closure of the used and folded-up/rolled-up pad adjacent to the edge of the pad. Positioning of the securing flap next to the edge of the pad is compatible with the most commonly encountered method of folding together/rolling up a pad after use.

### BRIEF DESCRIPTION OF THE FIGURES

- Figure 1: shows a pad in accordance with a preferred embodiment of the invention from the side that is intended to face towards the object that is to be protected
- Figure 2a: shows a section through the means in Figure 1 for closing the pad before the means has been used
- Figure 2b: shows a section through the means in Figure 2a in the outstretched state before it is to be closed against the backing layer of the pad
- Figure 2c: shows a section through the means in Figure 2b when the means has been secured to the back of the pad
- Figure 3: shows a rolled-up/folded pad before the means of closure has been used
- Figure 4: shows the pad in Figure 3 after closure with the help of the means
- Figure 5a: shows a section through an alternative means for closing the pad before the means has been used
- Figure 5b: shows a section through the means in Figure 5a when the means is secured to the back of the pad

### DESCRIPTION OF THE ILLUSTRATIVE EMBODIMENTS

The invention relates to a pad for disposable use comprising means for the closure of the pad in a folded configuration that is suitable for disposal after use.

Pads of the type described here have many different areas of application in institutional care, care in the home and the care of infants who are not yet potty trained.
Pads are used first and foremost to protect bedclothes and mattresses in conjunction with the care of incontinent patients, although the protection of seat cushions, wheelchairs or the like is also a common occurrence. Changing tables, on which diapers are changed on infants, are another surface that it is customary to protect with the help of different types of pad.

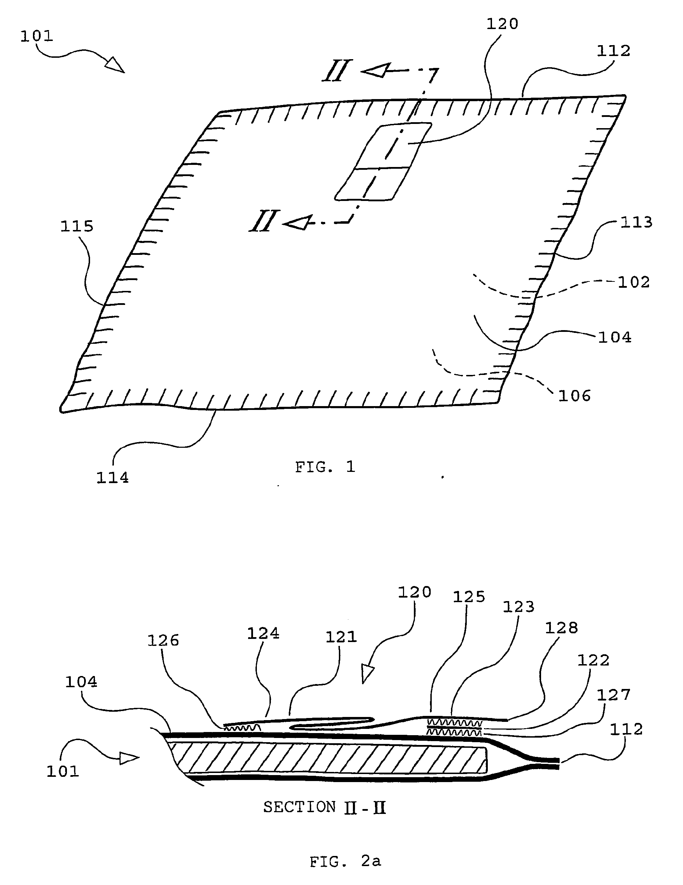

Figure 1 shows essential components of a pad 101 in accordance with the invention.
The pad 101 is essentially rectangular and exhibits four edges 112,113,114,115.

The pad 101 comprises a liquid-receiving covering layer 102, arranged over the surface of the pad 101 that is intended to face towards the user during use. The pad 101 further exhibits a backing layer 104 arranged over the surface of the pad that is intended during use to face towards the object that the pad is intended to protect. The pad also comprises an absorption body 106 enclosed between the water-receiving covering layer 102 and the backing layer 104. Because the pad 101 comprises a separate absorption body 106, the primary function of the water-receiving covering layer 102 is to allow liquid to pass through, as a consequence of which the absorbent capacity of the liquid-receiving layer 102 is of no significance or only secondary significance.

The liquid-receiving covering layer 102 of the pad 101 extends outside the absorption body 106 along the entire periphery of the absorption body 106. The liquid-receiving covering layer 102 can consist of any material that is suitable for the purpose. Examples of commonly encountered liquid-receiving covering materials are non-woven textile materials, known as nonwoven materials, perforated plastic films, meshes made of plastic or textile, and liquid-permeable foam layers. Liquid-receiving covering materials that are made of continuous thin fibers, which extend over the entire pad, are also conceivable. Laminates consisting of two or more of the above-mentioned possible covering materials are also commonly encountered, as are coverings consisting of different materials in different parts of the surface.

Pads 101 comprising absorption bodies 106 which exhibit especially high strength and resistance to wear may even function without the need to provide any extra liquid-receiving covering layer on the side of the pad 101 that faces towards the user during use.

Certain alternative pads exhibit no special absorption body, but the liquid-receiving covering layer consists of a combined covering and absorption layer. A common feature of such layers of material is that they contain natural fibers, for example cotton fibers, or rayon fibers. Pads that are constructed without a separate absorption body are intended primarily for situations in which larger absorption quantities are not anticipated.

The backing layer 104 also extends outside the absorption body 106 along the entire periphery of the absorption body 106. Backing layers 104 that are normally present on pads 101 are usually liquid-impermeable, although other types of backing layer can also be envisaged. The backing layer 104 can consist of a range of different materials. It is most common for the backing layer 104 to consist of a thin, liquid-tight plastic film, although it is also possible to use other types of liquid-tight materials such as nonwoven materials that have been made liquid-tight, for example by means of plastic coating, liquid-tight foam layers, liquid-tight adhesive or similar. The backing layer 104 can also consist of a liquid-tight, vapor-permeable material, as a consequence of which the pad 101 exhibits a certain capacity to breathe, which makes the pad more comfortable in use. Also encountered are laminates containing at least one liquid-tight layer usually arranged against the absorption body 106. Such laminates normally consist of a liquid-tight material functioning as a moisture barrier and a more textile-like material arranged on the side of the pad 101 that faces towards the object that is to be protected. Pads which exhibit such a backing layer exhibit greater friction with the object that is to be protected compared with a pad with a plastic back, for example, as a consequence of which the tendency to come out of position is reduced during use. The textile-like layer of the laminate usually consists of a nonwoven layer, although it can naturally also consist of other textile materials.

In order to ensure that the pad remains in place without sliding on the supporting surface (the object to be protected), pads also exist with adhesive surfaces and/or high-friction surfaces arranged in appropriate parts of the side of the pad that is intended to face towards the object that is to be protected.

The liquid-receiving covering layer 102 and the backing layer 104 are attached to one another outside the absorption body 106 along the entire periphery of the absorption body 106.

The liquid-receiving covering layer 102 and the backing layer 104 may be attached to one another by a number of different means. Examples of means of attachment include gluing, thermal fusion, ultrasonic welding or the like.

The pad 101 is characterized first and foremost in that it exhibits means 120 for closing the pad 101 after use.

Figure 2a shows the means 120 for closing as a sectioned view along the line II-II in Figure 1, in conjunction with which the means 120 is shown in a position before it is used.

The means 120 comprises an elongated securing flap 121, a protective layer 122 and a securing device 123. The securing flap 121 is arranged in conjunction with the edge 112 of the pad 101, as a consequence of which it is permanently attached to the backing layer 104 adjacent to its first end area 124. The second end area 125 of the securing flap 121 is attached in a detachable fashion to the backing layer 104 of the pad 101 via a protective layer 122 that has been treated with a release agent.
The protective layer 122 is treated with a release agent on the side that faces towards the securing flap 121, whereas the opposite side of the protective layer 122 is not treated with a release agent. The side of the protective layer 122 that is not treated with a release agent is permanently attached to the backing layer 104 of the pad 101 via the glued joint 127. The side of the protective layer 122 that is treated with a release agent is attached in a detachable fashion to the securing flap 121 via the securing device 123, in conjunction with which the securing device 123 is permanently attached to the securing flap 121.

The protective layer 122 consists of a piece of material made of paper that is treated with a release agent by means of silicon on the side that faces towards the securing flap 121.

In alternative embodiments, the protective layer can consist of a plastic layer which strengthens the backing layer 104 of the pad 101 so that the adhesive surface of the securing device 123 can be detached from the backing layer 104 of the pad 101 without the backing layer tearing in spite of the relatively high parting force. Plastic layers that are embossed are preferable because the active surface of the glued joint is reduced, as a consequence of which the parting force is reduced.

The securing device 123 preferably consists of an adhesive surface with the ability to adhere to the majority of commonly encountered materials on pads 101, and especially to a plastic film surface, which is the baking layer material most commonly encountered in a pad.

The securing device 123 does not extend as far as the front edge 128 of the securing flap 121, which makes it easier to take hold of the securing flap 121 when detaching it from the protective layer 122.

The securing flap 121 is folded in the form of a Z in its initial state, in conjunction with which the front edge 128 of the securing flap 121 is arranged close to the edge 112 of the pad 101, so that the second end area 125 of the securing flap 121 extends past the edge 112 of the pad 101 when the Z-folded part is stretched out. The securing flap 121 consists of a strip of paper, although in alternative embodiments it can consist of a strip of nonwoven material, plastic or the like.
Different types of laminate are also conceivable as securing flaps.
On the second end area 125 of the securing flap 121, the securing device 123 is arranged on the side of the securing flap 121 that faces towards the backing layer 104 of the pad 101.

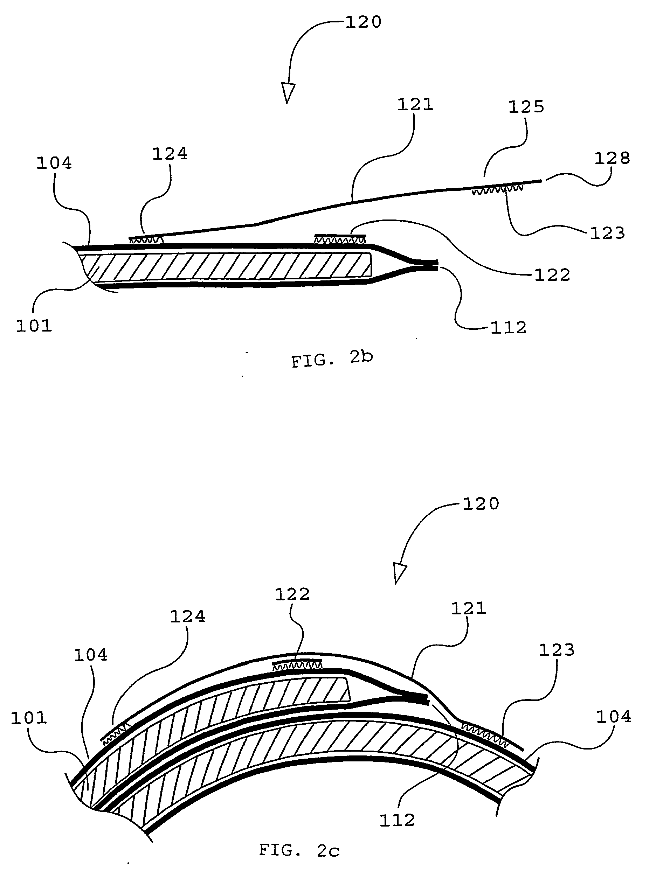

Figure 2b shows the means 120 in its detached state before closure against the backing layer 104 of the pad 101 has been effected. The second end area 125 of the securing flap 121 comprising the securing device 123 now constitutes the free end of the securing flap 121. The Z-folding of the securing flap 121 has now been stretched out so that the securing flap 121 extends past the edge 112 of the pad 101. The securing flap 121 has not yet been secured to the pad 101. The securing device 123 faces towards the pad 101.

Figure 2c shows a section through the means 120 in Figure 2b when the securing flap 121 is secured to the backing layer 104 of the pad 101, and the part of the pad 101 to which the securing flap 121 has been attached. The pad 101 is folded up/rolled up, as a consequence of which the backing layer 104 of the pad 101 faces outwards, and to which the securing flap 121 has been attached. The attachment created between the backing layer 104 of the pad 101 and the securing device 123 is essentially permanent, because the backing layer 104 of the pad has not been treated with a release agent where the attachment is arranged.

A tight and hygienic unit has been created, which is easy to handle/dispose of even if it contains urine or feces.

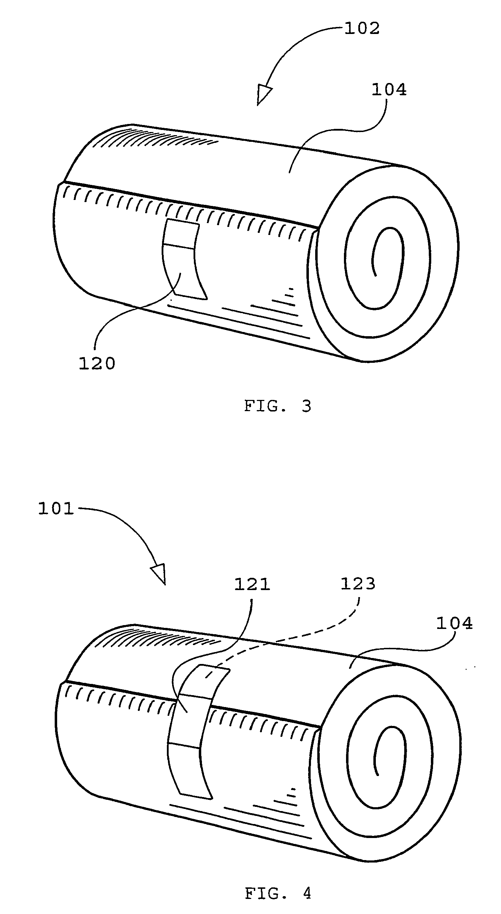

Figure 3 shows the pad 101 in Figure 1 after it has been rolled up with the backing layer 104 facing outwards to the surroundings. The means 120 for closing the pad has not yet been used to close the pad 101.

In Figure 4, the means 120 for closing the pad 101 has been taken into account, in conjunction with which the securing device 123 has been attached to the backing layer 104 so that the pad 101 is held together by the securing flap 121. The fold indications which derive from the Z-folding of the securing flap 121 are shown in the form of transverse markings on the securing flap 121.

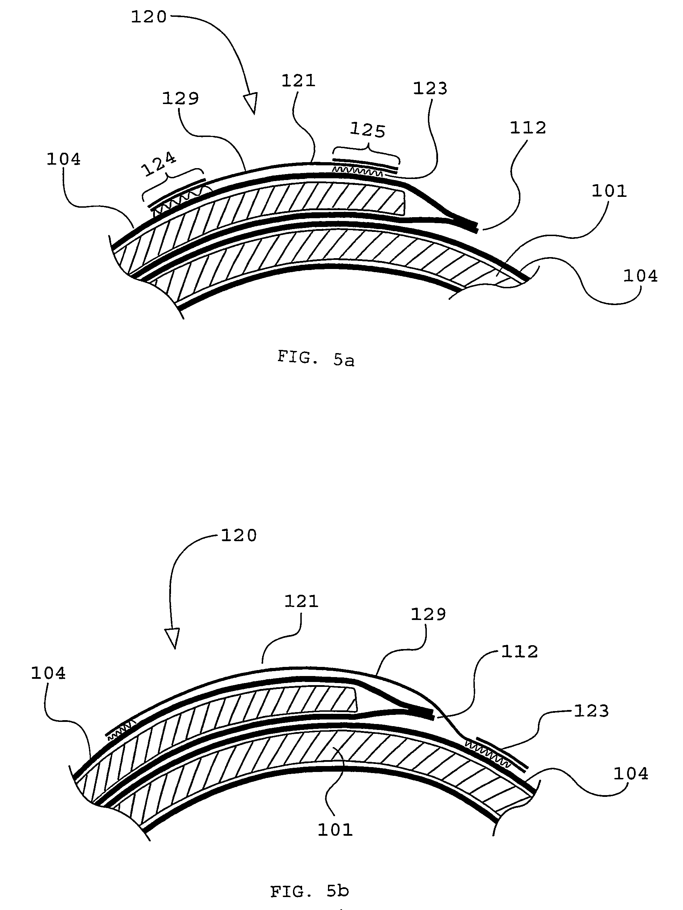

Shown in Figure 5a is an alternative embodiment of a means 120 for closing the pad 101, and the part of the pad 101 to which the means 120 is intended to be attached.
The securing flap 121 in this embodiment exhibits an elastic area 129 between the first and the second end area 124,125, in conjunction with which the securing flap 121 is capable of being extended in the longitudinal direction of the securing flap 121. The elastic securing flap 121 can be extended so that the second end area 125 of the securing flap 121 passes the edge 112 of the pad. The securing flap 121 thus does not need to be folded in its initial configuration in order for the second end area 125 of the securing flap 121 to be capable of extending beyond the edge 112 of the pad 101.
The end areas 124,125 of the securing flap 121, that is to say the areas where the securing flap 121 is attached to the backing layer 104 of the pad 101, exhibit no elastic properties. One reason for this is that it is comparatively more complicated to attach an elastic material, than a non-elastic material, to another material, although it is naturally also possible for the end areas 124,125 of the securing flap 121 to consist of an elastic material.
The end area 124 of the securing flap 121 is permanently attached to the backing layer of the pad 101, while the second end area 125 is attached in a detachable fashion to the backing layer 104. The backing layer 104 in this case is treated locally with a release agent under the securing device 123 which is not in accordence with the invention, as a consequence of which no separate protective layer is required in this embodiment.

Figure 5b shows how a folded-up/rolled-up pad 101 has been closed after use with the backing layer 104 facing outwards. The liquid-tight backing layer 104 constitutes an effective barrier against any matter excreted from the body contained in the pad 101. The elastic area 129 of the securing flap 121 has been extended past the edge 112 of the pad before it has been attached to the backing layer 104 of the pad 101. The new connection between the backing layer 104 and the securing device 123 may be regarded as permanent, since the backing layer is not treated with a release agent where the connection is arranged.

The invention also extends to all conceivable combinations of the described illustrative embodiments.

Furthermore, the invention is not restricted to the above-mentioned illustrative embodiments, but is naturally applicable to other embodiments within the scope of the following patent claims.

## Claims

1. A pad (101) of the disposable type for the protection of objects that require to be protected, for example seat cushions, bedclothes or changing tables, comprising a liquid-receiving covering layer (102) intended to face away from the object that requires to be protected, an essentially liquid-tight backing layer (104) intended to face towards the object that requires to be protected when it is being used, in conjunction with which the pad (101) also comprises means (120) for closing the pad (101) after use,
**CHARACTERIZED IN THAT**
the means (120) comprises at least one securing flap (121) comprising a first end area (124) and a second end area (125), in conjunction with which the first end area (124) is permanently attached to the backing layer (104) or to the liquid-receiving covering layer (102), in conjunction with which the second end area (125) of the securing flap (121) comprises a securing device (123) intended to be secured to the pad (101) after use and a protective layer (122) is arranged between the securing device (123) and the backing layer (104) or the liquid-receiving covering layer (102), in conjunction with which the protective layer (122) is treated with a release agent on the side which faces towards the securing device (123), and in conjunction with which the opposite side of the protective layer (122) is permanently attached to the backing layer (104) or to the liquid-receiving covering layer (102).

2. A pad (101) as claimed in claim 1,
**CHARACTERIZED IN THAT**
the pad (101) comprises an absorbent layer (106) arranged between the liquid-receiving covering layer (102) and the backing layer (104).

3. A pad (101) as claimed in claims 1 or 2,
**CHARACTERIZED IN THAT**
the securing device (123) is arranged on the side of the securing flap (121) which faces towards the pad (101).

4. A pad (101) as claimed in one or other of the foregoing claims,
**CHARACTERIZED IN THAT**
the securing device (123) comprises an adhesive surface.

5. A pad (101) as claimed in one or other of the foregoing claims,
**CHARACTERIZED IN THAT**
the means (120) for closing the pad (101) is arranged on the backing layer (104) of the pad.

6. A pad (101) as claimed in one or other of the foregoing claims,
**CHARACTERIZED IN THAT**
the securing flap (121) consists of a paper strip, in conjunction with which the paper strip includes a folded area, in conjunction with which the securing flap (121) can be extended by stretching out the folded area.

7. A pad (101) as claimed in one or other of claims 1-5,
**CHARACTERIZED IN THAT**
the securing flap (121) comprises an elastic area (129), in conjunction with which the securing flap (121) can be extended by stretching out the elastic area (129).

8. A pad (101) as claimed in claims 6 or 7,
**CHARACTERIZED IN THAT**
the second end area (125) of the securing flap (121) is arranged between the first end area (124) of the securing flap (121) and the edge (112) of the pad (101) when the securing flap (121) is in its first folded or unstretched state, and in conjunction with which the second end area (125) of the securing flap (121) reaches beyond the edge (112) of the pad when the fold is unfolded or when the elastic area (129) is stretched out.

## Patentansprüche

1. Unterlage (101) des Einwegtyps zum Schutz von Gegenständen, die geschützt werden müssen, z.B. Sitzkissen, Bettzeug oder Wickeltische, umfassend eine flüssigkeitsaufnehmende Deckschicht (102), die vom zu schützenden Gegenstand abwenden soll, eine im Wesentlichen flüssigkeitsdichte Unterschicht (104), die dem zu schützenden Gegenstand zugewandt sein soll, wenn sie benutzt wird, wobei die Unterlage (101) auch Mittel (120) zum Schließen der Unterlage (101) nach der Anwendung umfasst, **dadurch gekennzeichnet, dass** das Mittel (120) mindestens eine Befestigungslasche (121) mit einem ersten Endbereich (124) und einem zweiten Endbereich (125) umfasst, wobei der erste Endbereich (124) mit der Unterschicht (104) oder mit der flüssigkeitsaufnehmenden Deckschicht (102) fest verbunden ist, wobei der zweite Endbereich (125) der Befestigungslasche (121) eine Befestigungsvorrichtung (123) umfasst, die eingerichtet ist, um an der Unterlage (101) nach der Anwendung befestigt zu werden, und eine Schutzschicht (122) zwischen der Befestigungsvorrichtung (123) und der Unterschicht (104) oder der flüssigkeitsaufnehmenden Deckschicht (102) angeordnet ist, wobei die Schutzschicht (122) mit einem Trennmittel auf der Seite, die der Befestigungsvorrichtung (123) zugewandt ist, behandelt worden ist, und wobei die gegenüberliegende Seite der Schutzschicht (122) mit der Unterschicht (104) oder mit der flüssigkeitsaufnehmenden Deckschicht (102) fest verbunden ist.

2. Unterlage (101) nach Anspruch 1, **dadurch gekennzeichnet, dass** die Unterlage (101) eine saugfähige Schicht (106) umfasst, die zwischen der flüssigkeitsaufnehmenden Deckschicht (102) und der Unterschicht (104) angeordnet ist.

3. Unterlage (101) nach Anspruch 1 oder 2, **dadurch gekennzeichnet, dass** die Befestigungsvorrichtung (123) auf der Seite der Befestigungslasche (121), die der Unterlage (101) zugewandt ist, angeordnet ist.

4. Unterlage (101) nach irgendeinem der vorhergehenden Ansprüche, **dadurch gekennzeichnet, dass** die Befestigungsvorrichtung (123) eine Klebestelle umfasst.

5. Unterlage (101) nach irgendeinem der vorhergehenden Ansprüche,
**dadurch gekennzeichnet, dass** das Mittel (120) zum Schließen der Unterlage (101) an der Unterschicht (104) der Unterlage angeordnet ist.

6. Unterlage (101) nach irgendeinem der vorhergehenden Ansprüche,
**dadurch gekennzeichnet, dass** die Befestigungslasche (121) aus einem Papierstreifen besteht, wobei der Papierstreifen einen gefalteten Bereich umfasst, wobei die Befestigungslasche (121) durch Ausziehen des gefalteten Bereichs verlängert werden kann.

7. Unterlage (101) nach irgendeinem der Ansprüche 1-5 ,
**dadurch gekennzeichnet, dass** die Befestigungslasche (121) einen elastischen Bereich (129) umfasst, wobei die Befestigungslasche (121) durch Ausziehen des elastischen Bereichs (129) verlängert werden kann.

8. Unterlage (101) nach Anspruch 6 oder 7, **dadurch gekennzeichnet, dass** der zweite Endbereich (125) der Befestigungslasche (121) zwischen dem ersten Endbereich (124) der Befestigungslasche (121) und der Kante (112) der Unterlage (101) angeordnet ist, wenn die Befestigungslasche (121) im ersten gefalteten der unausgezogenen Zustand ist, und wobei sich der zweite Endbereich (125) der Befestigungslasche (121) über die Kante (112) der Unterlage hinaus erstreckt, wenn die Falte aufgefaltet ist, oder wenn der elastische Bereich (129) ausgezogen ist.

## Revendications

1. Matelas (101) du type jetable pour la protection des objets nécessitant d'être protégés, par exemple des coussins de siège, la literie ou des tables à changer, comprenant une couche à recouvrement pour réception de liquide (102) destinée à être détournée de l'objet nécessitant d'être protégé, une couche de support essentiellement étanche aux liquides (104) destinée à être tournée vers l'objet nécessitant d'être protégé une fois qu'il est utilisé, le matelas (101) comprenant en outre un moyen (120) pour fermer le matelas (101) après utilisation,
**caractérisé en ce que**
le moyen (120) comprend au moins un rabat de fixation (121) comprenant une première zone d'extrémité (124) et une deuxième zone d'extrémité (125), la première zone d'extrémité (124) étant fixée de manière permanente à la couche de support (104) ou à la couche à recouvrement pour réception de liquide (102), la deuxième zone d'extrémité (125) du rabat de fixation (121) comprenant un dispositif de fixation (123) destiné à être fixé au matelas (101) après l'utilisation, et une couche de protection (122) est disposée entre le dispositif de fixation (123) et la couche de support (104) ou la couche à recouvrement pour réception de liquide (102), la couche de protection (122) étant traitée avec un agent de libération sur le côté qui est orienté vers le dispositif de fixation (123), et le côté opposé de la couche de protection (122) étant fixé de manière permanente à la couche de support (104) ou à la couche à recouvrement pour réception de liquide (102).

2. Matelas (101) selon la revendication 1,
**caractérisé en ce que**
le matelas (101) comprend une couche absorbante (106) disposée entre la couche à recouvrement pour réception de liquide (102) et la couche de support (104).

3. Matelas (101) selon la revendication 1 ou 2,
**caractérisé en ce que**
le dispositif de fixation (123) est disposé sur le côté du rabat de fixation (121) qui est orienté vers le matelas (101).

4. Matelas (101) selon l'une ou l'autre des revendications précédentes,
**caractérisé en ce que**
le dispositif de fixation (123) comprend une surface adhésive.

5. Matelas (101) selon l'une ou l'autre des revendications précédentes,
**caractérisé en ce que**
le moyen (120) pour fermer le matelas (101) est disposé sur la couche de support (104) du matelas.

6. Matelas (101) selon l'une ou l'autre des revendications précédentes,
**caractérisé en ce que**
le rabat de fixation (121) est constitué d'une bande de papier, la bande de papier comprenant une zone pliée, le rabat de fixation (121) pouvant être étendu en allongeant la zone pliée.

7. Matelas (101) selon l'une ou l'autre des revendications 1 à 5,
**caractérisé en ce que**
le rabat de fixation (121) comprend une zone élastique (129), le rabat de fixation (121) pouvant être étendu en allongeant la zone élastique (129).

8. Matelas (101) selon les revendications 6 ou 7,
**caractérisé en ce que**
la deuxième zone d'extrémité (125) du rabat de fixation (121) est disposée entre la première zone d'extrémité (124) du rabat de fixation (121) et le bord (112) du matelas (101) lorsque le rabat de fixation (121) est dans son premier état plié ou non étiré, et la deuxième zone d'extrémité (125) du rabat de fixation (121) s'étendant au-delà du bord ( 112) du matelas lorsque le repli est non plié ou lorsque la zone élastique (129) est allongée.
